(19) Europäisches Patentamt
European Patent Office
Office européen des brevets

(11) Veröffentlichungsnummer: **0 528 203 B1**

# (12) EUROPÄISCHE PATENTSCHRIFT

(45) Veröffentlichungstag der Patentschrift: **11.10.95**

(51) Int. Cl.[6]: **C07C 233/20**, C08F 20/58, G03F 7/039

(21) Anmeldenummer: **92112588.6**

(22) Anmeldetag: **23.07.92**

## (54) Strahlungsempfindliches Gemisch mit einem polymeren Bindemittel mit Einheiten aus alpha,beta-ungesättigten Carbonsäureamiden.

(30) Priorität: **09.08.91 DE 4126409**

(43) Veröffentlichungstag der Anmeldung:
**24.02.93 Patentblatt 93/08**

(45) Bekanntmachung des Hinweises auf die Patenterteilung:
**11.10.95 Patentblatt 95/41**

(84) Benannte Vertragsstaaten:
**DE FR GB IT**

(56) Entgegenhaltungen:
**EP-A- 0 347 660**

(73) Patentinhaber: **HOECHST AKTIENGESELL-SCHAFT**

**D-65926 Frankfurt am Main (DE)**

(72) Erfinder: **Röschert, Horst, Dr.**
**Am Pfingstborn 16**
**W-6531-Ober Hilbersheim (DE)**
Erfinder: **Pawlowski, Georg, Dr.**
**Fritz-Kalle-Strasse 34**
**W-6200 Wiesbaden (DE)**
Erfinder: **Przybilla, Klaus-Jürgen, Dr.**
**Beethovenstrasse 19**
**W-6000 Frankfurt am Main 1 (DE)**

EP 0 528 203 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein strahlungsempfindliches Gemisch, das ein polymeres Bindemittel mit säurespaltbaren Seitengruppen und eine bei Bestrahlung eine starke Säure bildende Verbindung enthält.

Die Erfindung betrifft daneben $\alpha,\beta$-ungesättigte Carbonsäureamide, mit denen die als Bindemittel verwendeten Polymere hergestellt werden können.

Ein vielfach verwendetes positiv arbeitendes strahlungsempfindliches Gemisch zur Herstellung strahlungsempfindlicher Aufzeichnungsmaterialien enthält ein o-Chinondiazid-Derivat und ein in wäßrig-alkalischen Lösemitteln lösliches Bindemittel, z. B. einen Novolak oder ein Polyhydroxystyrol. Die Empfindlichkeit dieser Systeme gegenüber Strahlung, insbesondere kurzwelliger Strahlung, ist jedoch teilweise unbefriedigend. Durch ihre hohe intrinsische Absorption sind Novolake als Bindemittel in einem Einlagen-Resistmaterial für die Deep-UV-Lithographie (220-330 nm) ungeeignet. Polyhydroxystyrole weisen demgegenüber günstigere Absorptionseigenschaften auf und zeichnen sich zusätzlich durch einen höheren Wärmestand aus. Jedoch sind Polymere dieses Typs nur durch aufwendige mehrstufige Synthesen zugänglich. Die lithographischen Eigenschaften von Polymeren aus unsubstituiertem Hydroxystyrol sind wegen ihrer unausgewogenen Hydrophilie/Hydrophobie-Bilanz sowohl in 3-Komponenten- als auch in 2-Komponenten-Systemen unbefriedigend. Daher besteht ein Bedarf an Bindemitteln für hochauflösende, hochempfindliche Resistmaterialien mit guter Transparenz auch im UV-2-Bereich sowie mit hohem Wärmestand, die wäßrig-alkalisch entwickelbar sind.

Gemische, die das Prinzip der sogenannten "chemischen Verstärkung" nutzen, d.h. die neben einer durch Säure spaltbaren Verbindung eine Verbindung enthalten, die unter Einwirkung von Strahlung eine katalytisch wirkende Säure freisetzt, zeigen allgemein eine höhere Strahlungsempfindlichkeit. Die säurebildenden Verbindungen sind z. B. Diazonium-, Phosphonium-, Sulfonium- und Iodoniumsalze, Nitrobenzylester, phenolische Methansulfonate, Diazo- und Halogenverbindungen. Die Verwendung der Oniumsalze als photochemische Säurebildner in Resistmaterialien ist z. B. aus der US-A 4,491,628 bekannt. Einen Überblick über die Anwendung von Oniumsalzen in Resistmaterialien gibt Crivello in Org. Coatings and Appl.Polym.Sci., 48, p. 65-69 (1985).

Strahlungsempfindliche Gemische aus Polymeren mit säurelabilen Seitengruppen und photochemischen Säurebildnern sind aus US-A 4,491,628 und FR-A 2,570,844 bekannt. Konkret offenbart sind in den beiden genannten Schriften allerdings nur Polymere aus para-substituiertem Styrol oder $\alpha$-Alkyl-styrol. Als säurelabile para-Substituenten sind allein tert.-Butoxycarbonyloxy- und Trialkylsilanyloxy-Gruppen aufgeführt. Die polymeren Bindemittel mit den säurelabilen Seitengruppen sind äußerst hydrophob. Die bei der Belichtung gebildete Säure bewirkt die Abspaltung der säurelabilen Schutzgruppen und läßt die polymeren Bindemittel dadurch alkalilöslich werden.

Copolymere, deren säurelabile Gruppen über ein phenolisches Sauerstoffatom gebunden sind, beispielsweise Copolymere aus p-Hydroxystyrol und p-(tert.-butoxycarbonyloxy)-styrol, sind aus J.Poly. Sci., Part A, Polym. Chem. Ed., Vol. 24, 2971-2980 (1986) bekannt.

Die in der DE-A 41 20 172 beschriebenen Polymere zeigen eine deutliche Verbesserung der Transparenzeigenschaften im gewünschten Wellenlängenbereich. Sie wird aber nur dadurch erreicht, daß ein hoher Anteil an säurelabilen Schutzgruppen eingeführt wird, wodurch man die Hydrophobie/Hydrophilie-Bilanz jedoch negativ beeinflußt. Ferner gibt es bei Systemen, die stark hydrophobe Bindemittel enthalten, in der Regel Haftungsprobleme.

Aufgabe der vorliegenden Erfindung ist es, ein neues, im kurzwelligen UV-Bereich hoch strahlungsempfindliches Gemisch für die Herstellung von Reliefstrukturen bereitzustellen, das sich mit wäßrig-alkalischen Lösungen entwickeln läßt.

Gelöst wird die Aufgabe durch ein strahlungsempfindliches Gemisch, das ein polymeres Bindemittel mit säurespaltbaren Seitengruppen und eine bei Bestrahlung eine starke Säure bildende Verbindung enthält.

Gegenstand der Erfindung ist demgemäß ein strahlungsempfindliches Gemisch, das dadurch gekennzeichnet ist, daß es

ein Polymer mit Einheiten der allgemeinen Formel (I)

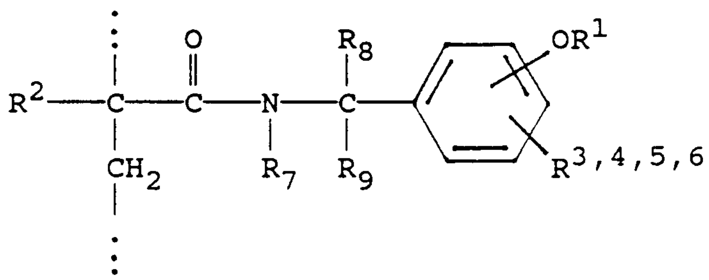

(I)

enthält, worin

R[1] eine durch Säure abspaltbare Gruppe und

R[2] $(C_1\text{-}C_4)$Alkyl, Halogen$(C_1\text{-}C_4)$alkyl, Halogen, CN oder Wasserstoff bedeutet,

$R^{3,4,5,6}$ voneinander unabhängig einen gegebenenfalls halogen-substituierten aliphatischen, araliphatischen oder aromatischen Rest mit 1 bis 20 Kohlenstoffatomen, Halogen, Hydroxy oder Wasserstoff darstellen und

R[7] ein Wasserstoffatom oder eine $(C_1\text{-}C_4)$Alkylgruppe bedeutet,

R[8] und R[9] gleich oder verschieden sind und Wasserstoff, $(C_1\text{-}C_6)$Alkyl- oder $(C_6\text{-}C_{10})$Arylgruppen darstellen.

Das erfindungsgemäße Gemisch zeigt im Vergleich zu den bekannten Gemischen mit Poly(4-hydroxystyrol), Novolaken oder Polymeren mit Einheiten aus Estern von $\alpha,\beta$-ungesättigten Carbonsäuren als Bindemittel folgende Vorteile:

a) die monomeren $\alpha,\beta$-ungesättigten Carbonsäure-amide lassen sich mit guter Ausbeute nach bewährten Verfahren (s. z.B. EP-A 0 347 660) aus preisgünstigen Ausgangsmaterialien herstellen,

b) für die Synthese der $\alpha,\beta$-ungesättigten Carbonsäureamide stehen zwei Synthesewege zur Verfügung, was verfahrenstechnische Vorteile bietet,

c) der Wärmestand der erfindungsgemäßen Polymere ist durch die Natur der Amid-Bindung und die daraus resultierenden Wasserstoffbrückenbindungen wesentlich höher als der von Polymeren aus den entsprechenden Estern,

d) leichte Zugänglichkeit der geschützten Monomere,

e) Stabilität der ungeschützten Monomere,

f) es lassen sich Homo- und Copolymere mit hohen Molekulargewichten aus den geschützten wie auch aus den ungeschützten Monomeren herstellen,

g) gute Transparenz im Wellenlängenbereich zwischen 220 und 300 nm,

h) ausgewogene Hydrophilie/Hydrophobie-Bilanz und

i) gute Haftungseigenschaften.

Polymere mit Einheiten der allgemeinen Formel I sind prinzipiell auf zwei verschiedenen Wegen herstellbar.

Zum einen können sogenannte "geschützte" Monomere der allgemeinen Formel II

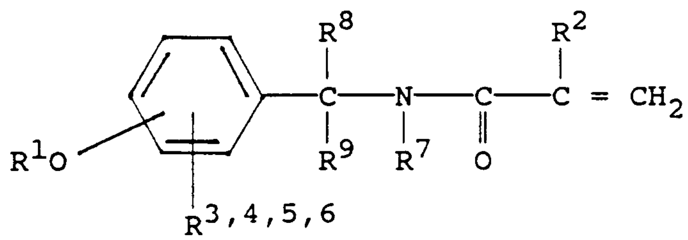

(II)

worin R[1] bis R[9] die oben angegebene Bedeutung haben, entweder allein oder mit anderen Monomeren polymerisiert werden. Diese Monomere sind neu und Teil der vorliegenden Erfindung. Als säurespaltbare Gruppe R[1] ist besonders Benzyl, Trialkylsilanyl, Alkoxycarbonyl (z.B. tert.-Butoxycarbonyl, Isopropoxycarbonyl und Pentyloxycarbonyl), Benzyloxycarbonyl, Tetrahydropyranyl oder Tetrahydrofuranyl geeignet.

Besonders bevorzugt sind Monomere der Formel II, in denen

R[1] eine Benzyl-, Trialkylsilanyl-, Alkoxycarbonyl-, Tetrahydropyranyl- oder Tetrahydrofuranyl-Gruppe,

$R^2$ Wasserstoff oder eine Methylgruppe ist,
$R^3$ bis $R^6$ unabhängig voneinander jeweils Wasserstoff, eine Hydroxy- oder eine Methylgruppe darstellen, wobei bevorzugt nicht mehr als zwei dieser Reste Methylgruppen sind, und
$R^7$ bis $R^9$ Wasserstoff bedeuten.

Ganz besonders bevorzugt als säurelabile Gruppe $OR^1$ ist die tert.-Butoxycarbonyloxygruppe; die Gruppe $OR^1$ steht vorzugsweise ortho- oder paraständig zu der $-CR^8R^9$-Gruppe.

Besonders bevorzugte Monomere sind mit säurelabilen Gruppen $R^1$ gebildete Derivate des N-(2- und 4-Hydroxybenzyl)-(meth)acrylamids, wie N-(4-tert.-butoxycarbonyloxy-benzyl)-(meth)acrylamid, N-(2-tert.-butoxycarbonyloxy-benzyl)-(meth)acrylamid, N-(3,5-Dimethyl-4-tert.-butoxycarbonyloxy-benzyl)-(meth)-acrylamid, N-(3-Methyl-4-tert.-butoxycarbonyloxy-benzyl)-(meth)acrylamid, N-(3-Methyl-2-tert.-butoxycarbonyloxy-benzyl)-(meth)acrylamid. Polymerisiert werden können auch Gemische dieser Monomere.

Zum anderen können die N-(Hydroxybenzyl)-(methacryl)amide polymerisiert werden, da diese in monomerer Form stabil sind. Die Polymere können dann in einem Folgeschritt derivatisiert werden, um die Gruppen $R^1$ einzuführen. Diese Verfahrensweise ist jedoch nicht bevorzugt, da sich die phenolischen Hydroxygruppen in den Polymeren nicht immer in reproduzierbarer Weise derivatisieren lassen. Zugleich ist es bei dem zweistufigen Verfahren unvermeidlich, daß Spuren der bei der Derivatisierungsreaktion verwendeten Base im Produkt verbleiben.

4-Hydroxystyrol ist dagegen in freier Form nicht beständig. Es läßt sich zudem nur in einer 4-Stufen-Synthese mit schlechten Ausbeuten herstellen. Deshalb kann als Ausgangsmaterial für die Polymerisation nur ein geschütztes 4-Hydroxystyrol, wie 4-(tert.-Butoxy-carbonyloxy)-styrol, verwendet werden. Letzteres wird über eine Wittig-Reaktion aus 4-Hydrory-benzaldehyd hergestellt (US-A 4,491,628). Alternativ dazu ist Poly(4-hydroxystyrol) mit geeigneten Schutzgruppen umgesetzt worden. Dieses Herstellungsverfahren hat jedoch den gravierenden Nachteil, daß dadurch das Bindemittel mit Metallionen und mit Base kontaminiert wird. Das Vorhandensein von Basen ist für photochemisch verstärkt arbeitende Systeme nachteilig. Eine hohe Metallionenkontamination ist für die Herstellung von Halbleitern sogar inakzeptabel. Nach diesem Verfahren hergestellte Bindemittel zeigen häufig nicht reproduzierbare lithographische Eigenschaften.

Diese Nachteile sind mit den erfindungsgemäßen Polymeren und dem damit hergestellten erfindungsgemäßen strahlungsempfindlichen Gemisch überwunden.

Die erfindungsgemäßen polymeren Bindemittel können sowohl Homopolymere sein, die ausschließlich Einheiten der allgemeinen Formel I enthalten, als auch allgemein Mischpolymere, die neben diesen noch andere Einheiten enthalten. Falls in den Polymeren neben den Einheiten der allgemeinen Formel I noch andere Einheiten vorhanden sind, so sind diese bevorzugt abgeleitet von "ungeschützten" und/oder säurestabil geschützten Monomeren der allgemeinen Formel II ($R^1$ ist in diesen Monomeren Wasserstoff bzw. ein durch Säure nicht abspaltbarer Rest). In diesen Polymeren können jedoch auch Einheiten vorkommen, die von anderen üblichen Vinylmonomeren abgeleitet sind. Als solche weiteren Monomere können besonders Ester oder Amide der (Meth)acrylsäure, wie Hydroxyethylmethacrylat (HEMA), aber auch Methacrylsäure selbst, Hydroxypropylmethacrylat, Methylmethacrylat, Ethylmethacrylat, Methacrylamid und Hydroxyethylmethacrylamid, fungieren. Die Haftungseigenschaften des Bindemittels lassen sich dadurch modifizieren.

Gemische mit erhöhter Plasmabeständigkeit werden dann erhalten, wenn zur Herstellung der Polymere Silicium enthaltende Monomere mitverwendet werden.

Schließlich sind darüber hinaus auch Mischpolymere mit Maleimid verwendbar, die in wäßrig alkalischen Lösungen eine erhöhte Löslichkeit zeigen und eine höhere Transparenz im Deep-UV-Bereich aufweisen. Den gleichen Effekt zeigen auch Copolymere mit Styrol, substituiertem Styrol, mit Vinylethern, Vinylestern, Vinylsilanverbindungen oder (Meth)acrylsäureestern.

Die erfindungsgemäßen Polymere enthalten mindestens 10 mol-% an Einheiten der allgemeinen Formel I. Ihr mittleres Molekulargewicht $M_n$ liegt zwischen 2.000 und 100.000, vorzugsweise zwischen 5.000 und 40.000 g/mol.

Das Bindemittel mit säurespaltbaren Gruppen ist im erfindungsgemäßen Gemisch im allgemeinen in Mengen von 45 bis 98 Gew.-%, vorzugsweise 85 bis 98 Gew.-%, bezogen auf das Gesamtgewicht der Feststoffe im strahlungsempfindlichen Gemisch, enthalten.

Das erfindungsgemäße Gemisch enthält weiterhin mindestens eine photoaktive Verbindung, die bei Bestrahlung eine starke Säure freisetzt, die ihrerseits die in dem Bindemittel vorhandene Schutzgruppen abspaltet und dadurch bewirkt, daß die Löslichkeit des Bindemittels und somit auch des Gemisches in wäßrig-alkalischer Lösung stark ansteigt. Das Gemisch ist besonders sensitiv gegenüber UV(220-400 nm)-, Elektronen- und Röntgenstrahlen und ist als Resistmaterial auch zur Herstellung von Druckplatten hervorragend geeignet.

Als Säurebildner kommen im Prinzip alle Verbindungen in Frage, die bei Bestrahlung eine starke Säure bilden. Beispiele besonders geeigneter Säurebildner sind Sulfoniumsalze der allgemeinen Formel $[(C_6H_5)_3S]^+X^-$, wobei X besonders für Chlorid, Bromid, Perchlorat, Hexafluorphosphat, Hexafluorarsenat, Hexafluorantimonat, Tetrafluorborat oder ein Sulfonat, wie Methansulfonat, Trifluormethansulfonat oder Toluol-4-sulfonat, steht. Geeignet sind auch Nitrobenzylester, Bissulfonyl-diazomethane, 1,2,3-Tris-(alkylsulfonyloxy- bzw. -arylsulfonyloxy)-benzol, 1-Sulfonyloxy-2-pyridone und Halogenverbindungen. Für die Bestrahlung mit kurzwelligen UV-Strahlen sind jedoch Jodonium- und besonders Sulfoniumsalze bevorzugt.

Der Säurebildner ist in dem Gemisch in einer Menge von 1 bis 40 Gew.-%, vorzugsweise 2 bis 15 Gew.-%, bezogen auf das Gesamtgewicht der Feststoffe in dem strahlungsempfindlichen Gemisch, vorhanden.

Das erfindungsgemäße strahlungsempfindliche Gemisch kann zusätzlich noch weitere übliche Hilfs- und Zusatzstoffe enthalten, wie Haftvermittler, Netzmittel, Farbstoffe und Weichmacher.

Gegebenenfalls können auch Sensibilisatoren in geringen Mengen zugesetzt werden, um den Säurebildner für Strahlung im längerwelligen UV- bis hin zum sichtbaren Bereich zu sensibilisieren. Polycyclische Aromaten, wie Pyren, Perylen und verschiedene Heteroaromaten, wie Phenothioazin, sind hierfür bevorzugt, jedoch können auch Farbstoffe, die als Sensibilisatoren wirken, verwendet werden.

Ferner betrifft die Erfindung ein Aufzeichnungsmaterial mit einem Träger und einer Schicht, die das erfindungsgemäße strahlungsempfindliche Gemisch enthält, sowie ein Verfahren zur Herstellung von Reliefstrukturen oder zur Strukturierung von Wafern.

Um das erfindungsgemäße Gemisch auf ein Trägermaterial auftragen zu können, wird es zweckmäßig in einem organischen Lösemittel gelöst, wobei der Feststoffgehalt im allgemeinen im Bereich zwischen 5 und 40 Gew.-% liegt. Als Lösemittel kommen bevorzugt aliphatische Ketone, Ether und Ester sowie beliebige Mischungen davon in Frage. Besonders bevorzugt sind Alkylenglykol-monoalkylether, wie beispielsweise Ethylcellosolve, Ethylenglykol-mono-butylether, Methylcellosolve und 1-Methoxy-2-propanol, Alkylenglykolalkylether-ester, wie z. B. Methylcellosolve-acetat, Ethylcellosolve-acetat, Propylenglykol-methylether-acetat und Propylenglykol-ethylether-acetat, Ketone, wie beispielsweise Cyclohexanon, Cyclopentanon und Butanon, sowie Acetate, wie Butylacetat, und Aromaten, wie Toluol und Xylol. Die Auswahl der entsprechenden Lösemittel bzw. deren Mischungen richtet sich nach der Wahl des jeweiligen phenolischen Polymers und der photoempfindlichen Komponente.

Bei dem erfindungsgemäßen Verfahren zur Herstellung von Reliefmustern wird eine strahlungsempfindliche Aufzeichnungsschicht, die im wesentlichen aus dem erfindungsgemäßen strahlungsempfindlichen Gemisch besteht, bildmäßig in solcher Dosis bestrahlt, daß die Löslichkeit der belichteten Bereiche in wäßrig-alkalischen Lösemitteln zunimmt und diese bestrahlten Bereiche dann selektiv mit dem alkalischen Entwickler entfernt werden können. Man enthält auf diese Weise ein positives Bild.

Ebenso ist auch eine Entwicklung mit organischen Lösemitteln wie Toluol oder Anisol möglich. Die unbelichteten Bereiche zeigen eine größere Lipophilie und werden daher abgetragen. Man erhält ein negatives Bild.

Das erfindungsgemäße strahlungsempfindliche Gemisch arbeitet je nach Wahl der Entwicklungsverfahren positiv oder negativ. Es eignet sich besonders als Photoresist zur Herstellung von Reliefstrukturen für Halbleiterbauelemente.

Die das erfindungsgemäße strahlungsempfindliche Gemisch enthaltenden Photoresistlösungen werden im allgemeinen in Schichten von 0,1 bis 5 µm, vorzugsweise 0,5 bis 1,5 µm auf geeignete Substrate, beispielsweise oberflächlich oxidierte Silicium-Wafer durch Aufschleudern (spin coating) aufgetragen, getrocknet (z. B. bei Temperaturen zwischen 70 und 130 °C) und mit einer geeigneten Lichtquelle durch eine Photomaske bildmäßig belichtet. Als Lichtquellen eignen sich insbesondere kurzwellige UV-Strahlen (deep UV) mit Wellenlängen zwischen 200 und 400 nm. Besonders geeignete Lichtquellen sind Excimer-Laser.

Nach dem bildmäßigen Belichten wird gegebenenfalls nach kurzem Ausheizen (post-bake) bei Temperaturen bis zu 150 °C mit üblichen wäßrig-alkalischen Entwicklerlösungen, im allgemeinen bei pH-Werten zwischen 12 und 14, entwickelt, wobei die belichteten Stellen ausgewaschen werden. Die Auflösung liegt im Subhalbmikron-Bereich. Die für das erfindungsgemäße strahlungsempfindliche Gemisch benötigte Belichtungsenergie liegt im allgemeinen zwischen 5 und 200 $mJ/cm^2$ bei Schichtdicken von 1 µm.

Die entwickelten Resiststrukturen werden gegebenenfalls nachgehärtet. Dies geschieht im allgemeinen dadurch, daß man die Resiststruktur auf einer hot-plate bis zu einer Temperatur unter der Fließtemperatur erhitzt und anschließend mit UV-Licht einer Xenon-Quecksilberdampflampe (Bereich 200 bis 250 nm) ganzflächig belichtet. Durch diese Nachhärtung werden die Resiststrukturen vernetzt, so daß die Strukturen eine Fließbeständigkeit im allgemeinen bis zu Temperaturen von über 200 °C aufweisen. Die Nachhärtung kann auch ohne Temperaturerhöhung unter Einstrahlung von UV-Licht erfolgen. Dies gilt insbesondere dann, wenn energiereiche Strahlung verwendet wird.

Bevorzugte Anwendung findet das erfindungsgemäße strahlungsempfindliche Gemisch in lithographischen Prozessen zur Herstellung integrierter Schaltungen oder von diskreten elektrischen Bausteinen. Das aus dem Gemisch hergestellte Aufzeichnunsmaterial dient dabei als Maske für die folgenden Prozeßschritte. Hierunter zählen z.B. das Ätzen des Schichtträgers, die Implantation von Ionen in den Schichtträger oder die Abscheidung von Metallen oder anderen Materialien auf den Schichtträger. Daneben ist das erfindungsgemäße strahlungsempfindliche Gemisch auch für die Herstellung von Druckplatten geeignet.

Die folgenden Beispiele erläutern die Herstellung der Monomeren, Homo- und Copolymeren sowie deren physikalische und lithographische Charakterisierung.

Die in den Beispielen angegebenen Teile und Prozente sind, soweit nicht anders angegeben, Gewichtsteile (Gt) bzw. Gewichtsprozente (Gew.-%).

Allgemeine Synthesevorschrift 1 zur Herstellung von N-(Hydroxy-benzyl)-(meth)acrylamid-Monomeren:

Die Herstellung erfolgt analog zu dem in der EP-A 0 347 660 angegebenen Verfahren. Dabei werden 129 g (1,0 mol) N-(Methoxymethyl)-(meth)acrylamid (MMMA), 1,0 mol des zur Umsetzung ausgewählten Phenols (Kresols, Xylenols etc.) und 0,1 mg (0,5 mmol) Phenothiazin in 150 ml Ethanol gelöst, mit 1,0 g (10 mmol) konzentrierter Schwefelsäure versetzt und 7 h zum Rückfluß erhitzt. Nach dem Abkühlen der Reaktionsmischung auf 5 °C kristallisiert das farblose Produkt aus. Es wird abfiltriert und im Vakuumtrokkenschrank bei 50 °C getrocknet.

Tabelle 1 enthält eine Reihe von bevorzugten Monomeren, die entsprechend der allgemeinen Synthesevorschrift 1 hergestellt werden. Weitere Beispiele können der CH-A 476 689, der DE-C 14 43 912 und der EP-A 0 347 660 entnommen werden.

Tabelle 1:

| Nr. | Monomere bzw. Monomerengemische | Ausbeute [%] |
|---|---|---|
| 1a | N-(4-Hydroxy-3,5-dimethyl-benzyl)-methacrylamid | 80 |
| 1b[a)] | (1c : 1d ≈ 2 : 1)[b)] | 92 |
| 1c[c)] | N-(4-Hydroxy-3-methyl-benzyl)-methacrylamid | --[d)] |
| 1d[c)] | N-(2-Hydroxy-3-methyl-benzyl)-methacrylamid | --[d)] |
| 1e[a)] | (1f : 1g ≈ 1 : 1)[b)] | 96 |
| 1f[c)] | N-(4-Hydroxy-benzyl)-methacrylamid | --[d)] |
| 1g[c)] | N-(2-Hydroxy-benzyl)-methacrylamid | --[d)] |
| 1h | N-(3,5-Diethyl-4-hydroxy-benzyl)-methacrylamid | 72 |
| 1i | N-(2-Hydroxy-3,5-dimethyl-benzyl)-methacrylamid | 75 |

a) Unter Verwendung von N-(Butoxymethyl)-(meth)acrylamid anstelle von MMMA hergestellt.

b) Die Isomerenverhältnisse werden durch Integration der Protonensignale in den $^{1}$H-NMR-Spektren bestimmt.

c) Die Monomeren werden aus den entsprechenden Monomergemischen (1c und 1d aus 1b, 1f und 1g aus 1e) mittels Säulenchromatographie an Kieselgel [Gradientenelution mit Petrolether/Essigester-Gemischen (3:1 ===> 1:7)] als Reinsubstanz isoliert.

d) Die isolierten Mengen stimmten mit den durch Integration der $^{1}$H-NMR-Signale erwarteten Werten überein.

Allgemeine Synthesevorschrift 2 zur Herstellung von mit tert.-Butoxycarbonyl-Gruppen geschützten N-(Hydroxybenzyl)-(meth)acrylamid-Monomeren:

Zu einer Lösung aus 0,15 mol eines N-(Hydroxybenzyl)-(meth)acrylamid-Monomeren oder eines Monomerengemisches 1 (entsprechend der in Tabelle 1 angegebenen Bedeutung) in 200 ml trockenem Tetrahydrofuran (THF) werden 0,1 mg (0,5 mmol) Phenothiazin und 30,0 g (0,22 mol) Kaliumcarbonat gegeben. Unter Rühren wird dann eine Lösung von 36,2 g (0,165 mol) Di-tert.-butyl-dicarbonat (Pyrokohlensäure-di-tert.-butylester) in 60 ml trockenem THF innerhalb von 30 min bei 5 bis 10 °C zugetropft. Nach dem Erwärmen auf Raumtemperatur rührt man noch weitere 40 h. Man gießt das Reaktionsgemisch auf 1,5 l Eiswasser, extrahiert mit Essigester, trocknet die organische Phase mit Natriumsulfat und destilliert das Lösemittel im Vakuum ab. Man erhält farblose, zähviskose Produkte, die nach längerem Stehen partiell kristallisieren und ohne weitere Reinigung für die nachfolgende Polymerisation verwendet werden. Einige der mit tert.-Butoxycarbonyl-Gruppen geschützten N-(Hydroxybenzyl)-(meth)-acrylamid-Monomere 2 können aus Isopropylether kristallisiert werden. Tabelle 2 enthält eine Reihe von bevorzugten Monomeren, die entsprechend der allgemeinen Synthesevorschrift 2 hergestellt werden.

Tabelle 2

| Nr. | Monomere bzw. Monomerengemische mit tert.-Butoxycarbonyl -Schutzgruppen | Ausbeute [%] |
|---|---|---|
| 2a | N-(4-tert.-Butoxycarbonyloxy-..... 3,5-dimethyl-benzyl)-methacrylamid | 89 |
| 2b | (2c : 2d ≈ 2 : 1) | 86 |
| 2c | N-(4-tert.-Butoxycarbonyloxy-..... 3-methyl-benzyl)-methacrylamid | 84 |
| 2d | N-(2-tert.-Butoxycarbonyloxy-3-methyl-benzyl)-methacrylamid | 71 |
| 2e | (2f : 2g ≈ 1 : 1) | 95 |
| 2f | N-(4-tert.-Butoxycarbonyloxybenzyl)-methacrylamid | 90 |
| 2g | N-(2-tert.-Butoxycarbonyloxybenzyl)-methacrylamid | 85 |
| 2h | N-(4-tert.-Butoxycarbonyloxy-3,5-diethyl-benzyl)-methacrylamid | 72 |
| 2i | N-(2-tert.-Butoxycarbonyloxy- -3,5-dimethyl-benzyl)-methacrylamid | 81 |

Allgemeine Synthesevorschrift 3 zur Herstellung von Homopolymeren aus N-(tert.-Butoxycarbonyloxybenzyl)-methacrylamiden:

20 g (etwa 60 mmol) des jeweiligen mit tert.-Butoxycarbonyl-Gruppen geschützten Monomers 2 (oder der Monomermischung 2) werden mit 0,30 g (1,83 mmol) Azobisisobutyronitril (AIBN) in 100 bis 150 ml trockenem Methylethylketon (MEK)/THF (1:2) (ggf. muß das MEK/THF-Verhältnis variiert werden bzw. Ethanol oder N,N-Dimethylformamid zugesetzt werden) 8 h unter Stickstoffatmosphäre zum Rückfluß erhitzt. Das Polymer wird in Petrolether ausgefällt und im Vakuumtrockenschrank bei 50 °C bis bis zur Gewichtskonstanz getrocknet.

Die Ausbeute bei allen Beispielen beträgt mehr als 90 %. Die Molekulargewichtsmittel $M_w$ liegen zwischen 18.000 und 24.000 g/mol, während die Zahlenmittel des Molekulargewichts $M_n$ um 13.000 g/mol ermittelt werden (durch Gelpermeationschromatographie bestimmt).

Nachfolgend sind einige der bevorzugten Homopolymeren aufgelistet, die entsprechend der allgemeinen Synthesevorschrift 3 hergestellt werden.

Polymer 3a:

Homopolymer aus N-(4-tert.-Butoxycarbonyloxy-3,5-dimethyl-benzyl)-methacrylamid

Polymer 3b:

Homopolymer aus N-(4-tert.-Butoxycarbonyloxy-benzyl)-methacrylamid

Polymer 3c:

Homopolymer aus N-(2-tert.-Butoxycarbonyloxy-benzyl)-methacrylamid

Polymer 3d:

Homopolymer aus N-(4-tert.-Butorycarbonyloxy-3-methylbenzyl)-methacrylamid

Polymer 3e:

Homopolymer aus N-(2-tert.-Butoxycarbonyloxy-3-methylbenzyl)-methacrylamid
Weiterhin werden eine Vielzahl verschiedener Co- und höherer Polymere entsprechend der allgemeinen Synthesevorschrift 3 hergestellt.
Die Copolymeren 4 erhält man aus den Monomermischungen 2b und 2e.

Polymer 4a:

1:1-Copolymer aus dem Monomerengemisch 2e [N-(4-tert.-Butoxycarbonyloxy-benzyl)-methacrylamid und N-(2-tert.-Butoxycarbonyloxy-benzyl)-methacryl-amid]

Polymer 4b:

2:1-Copolymer aus dem Monomerengemisch 2b [N-(4-tert.-Butoxycarbonyloxy-3-methyl-benzyl)-methacrylamid und N-(2-tert.-Butoxycarbonyloxy-3-methyl-benzyl)-methacrylamid]
Die Copolymeren 5 werden durch Umsetzung einer Mischung von N-(3,5-Dimethyl-4-tert.-butoxycarbonyloxy-benzyl)-methacrylamid 2a und N-(3,5-Dimethyl-4-hydroxy-benzyl)-methacryl-amid 1a in unterschiedlichen Monomerverhältnissen analog zu den Bedingungen der allgemeinen Synthesevorschrift 3 hergestellt.

Polymere 5a bis 5d:

Copolymere aus N-(3,5-Dimethyl-4-tert.-butoxycarbonyloxy-benzyl)-methacrylamid 2a (x) und N-(3,5-Dimethyl-4-hydroxy-benzyl)-methacryl-amid 1a (y).
Polymer 5a: x : y = 80 : 20
Polymer 5b: x : y = 60 : 40
Polymer 5c: x : y = 40 : 60
Polymer 5d: x : y = 20 : 80
Die Copolymeren 6 werden durch Umsetzung einer Mischung von N-(4-tert.-Butoxycarbonyloxy-3,5-dimethyl-benzyl)-methacrylamid 2a und Maleimid in unterschiedlichen Monomerverhältnissen analog zu den Bedingungen der allgemeinen Synthesevorschrift 3 hergestellt.

Polymere 6a bis 6e:

Copolymere aus N-(3,5-Dimethyl-4-tert.-butoxycarbonyloxy-benzyl)-methacrylamid 2a (x) und Maleimid (y).
Polymer 6a: x : y = 90 : 10
Polymer 6b: x : y = 80 : 20
Polymer 6c: x : y = 70 : 30
Polymer 6d: x : y = 60 : 40
Polymer 6e: x : y = 50 : 50
Die Polymere 7 und 8 werden durch Umsetzung einer Monomermischung aus 1b und 2b (Polymere 7) bzw. einer Monomermischung aus 1e und 2e (Polymer 8) in unterschiedlichen Mischungsverhältnissen analog zu den Bedingungen der allgemeinen Synthesevorschrift 3 hergestellt.

Polymere 7a bis 7d:

Polymere aus dem Monomerengemisch 1b [N-(4-Hydroxy-3-methyl-benzyl)-methacrylamid und N-(2-Hydroxy-3-methylbenzyl)-methacrylamid ≈ 2 : 1] (x) und dem Monomerengemisch 2b [N-(4-tert.-Butoxycarbonyloxy-3-methyl-benzyl)-methacrylamid und N-(2-tert.-Butoxycarbonyloxy-3-methylbenzyl)-methacrylamid ≈ 2 : 1] (y).
Polymer 7a: x : y = 10 : 90
Polymer 7b: x : y = 20 : 80
Polymer 7c: x : y = 30 : 70

Polymer 7d: x : y = 40 : 60

Polymere 8a bis 8d:

Polymere aus dem Monomerengemisch 1e [N-(4-Hydroxy-benzyl)-methacrylamid und N-(2-Hydroxy-benzyl)-methacrylamid ≈ 1 : 1] (x) und dem Monomerengemisch 2e [N-(4-tert.-Butoxycarbonyloxy-benzyl)-methacrylamid und N-(2-tert.-Butoxycarbonyloxy-benzyl)-methacryl-amid ≈ 1 : 1] (y).
Polymer 8a: x : y = 10 : 90
Polymer 8b: x : y = 20 : 80
Polymer 8c: x : y = 30 : 70
Polymer 8d: x : y = 40 : 60
Dabei zeigt sich, daß die im Polymer vorhandene Amidfunktion ausreichend ist, um gute Haftungseigenschaften zu erreichen.

Die folgenden Anwendungsbeispiele 1 bis 5 belegen die Eignung des erfindungsgemäßen Gemischs für Aufzeichnungsmaterialien in der Mikrolithographie. Die in diesen Beispielen eingesetzten 1-Sulfonyloxy-2-pyridone sowie Verfahren zu deren Herstellung sind in der DE-A 41 12 967 beschrieben.

Anwendungsbeispiel 1:

Es wird eine Lösung eines strahlungsempfindlichen Gemisches hergestellt, enthaltend
2,5 Gt des Copolymers 6c, hergestellt aus 70 % N-(4-tert.-Butoxycarbonyloxy-3,5-dimethyl-benzyl)-methacrylamid 2a und 30 % Maleimid, und
0,075 Gt 4-Methyl-6-styryl-1-trifluormethansulfonyloxy-2-pyridon und
7,5 Gt Propylenglykol-monomethylether-acetat.

2,0 ml dieser Lösung werden durch ein Filter mit einem Porendurchmesser von 0,2 µm filtriert und auf einen Siliciumwafer, der mit Hexamethyldisilazan als Haftvermittler überzogen ist, aufgetragen. Die Schleuderdrehzahl wird dabei so gewählt, daß man nach 40 s eine Schichtdicke von etwa 1 µm erhält. Der Wafer wird auf einer Heizplatte bei 100 °C 60 s lang getrocknet, anschließend mit einer bildmäßig strukturierten Testmaske in Kontakt gebracht und unter Verwendung einer Xenon-Quecksilberdampflampe und einem zwischengeschalteten Interferenzfilter mit Strahlung von 365 nm bestrahlt (65 mJ/cm$^2$). Danach wird der belichtete Wafer 1 min einem post exposure bake von 80 °C unterzogen und mit einer 0,05 n wäßrigen Tetramethylammoniumhydroxid-Lösung 75 s entwickelt. Strukturen von 0,60 µm (line/space) werden aufgelöst.

Anwendungsbeispiel 2:

Es wird eine Lösung eines strahlungsempfindlichen Gemisches hergestellt, enthaltend
2,5 Gt des Copolymers 5c, hergestellt aus 40 % N-(4-tert.-Butoxycarbonyloxy-3,5-dimethyl-benzyl)-methacrylamid 2a und 60 % N-(4-Hydroxy-3,5-dimethyl-benzyl)-methacrylamid 1a, und
0,075 Gt 4-Methyl-6-styryl-1-trifluormethansulfonyloxy-2-pyridon und
7,5 Gt Propylenglykol-monomethylether-acetat.

2,0 ml dieser Lösung werden durch ein Filter mit einem Porendurchmesser von 0,2 µm filtriert und auf einen Siliciumwafer, der mit Hexamethyldisilazan als Haftvermittler überzogen ist, aufgetragen. Die Schleuderdrehzahl wird dabei so gewählt, daß man nach 40 s eine Schichtdicke von etwa 1 µm erhält. Der Wafer wird auf einer Heizplatte bei 100 °C 60 s lang getrocknet, anschließend mit einer bildmäßig strukturierten Testmaske in Kontakt gebracht und unter Verwendung einer Xenon-Quecksilberdampflampe und einem zwischengeschalteten Interferenzfilter mit Strahlung von 365 nm bestrahlt (65 mJ/cm$^2$). Danach wird der belichtete Wafer 1 min einem post exposure bake von 80 °C unterzogen und mit einer 0,09 n wäßrigen Tetramethylammoniumhydroxid-Lösung 75 s entwickelt. Strukturen von 0,70 µm (line/ space) werden aufgelöst.

Anwendungsbeispiel 3:

Es wird eine Lösung eines strahlungsempfindlichen Gemisches hergestellt, enthaltend
2,5 Gt des 2:1-Copolymers 4b, hergestellt aus einem Monomerengemisch 2b [65 % N-(4-tert.-Butoxycarbonyloxy-3-methyl-benzyl)-methacrylamid und 35 % N-(2-tert.-Butoxycarbonyloxy-3-methylbenzyl)-methacrylamid, und
0,075 Gt Triphenylsulfonium-trifluormethansulfonat und

7,5 Gt Propylenglykol-monomethylether-acetat.

2,0 ml dieser Lösung werden durch ein Filter mit einem Porendurchmesser von 0,2 µm filtriert und auf einen Siliciumwafer, der mit Hexamethyldisilazan als Haftvermittler überzogen ist, aufgetragen. Die Schleuderdrehzahl wird dabei so gewählt, daß man nach 40 s eine Schichtdicke von etwa 1 µm erhält. Der Wafer wird auf einer Heizplatte bei 100 °C 60 s lang getrocknet, anschließend mit einer bildmäßig strukturierten Testmaske in Kontakt gebracht und unter Verwendung einer Xenon-Quecksilberdampflampe und einem zwischengeschalteten Interferenzfilter mit Strahlung von 365 nm bestrahlt (60 $mJ/cm^2$). Danach wird der belichtete Wafer 1 min einem post exposure bake von 85 °C unterzogen und mit einer 0,135 n wäßrigen Tetramethylammoniumhydroxid-Lösung 45 s entwickelt. Strukturen von 0,60 µm (line/space) werden aufgelöst.

Anwendungsbeispiel 4:

Es wird eine Lösung eines strahlungsempfindlichen Gemisches hergestellt aus
2,5 Gt des Polymeren 8a, entsprechend der oben angegebenen Zusammensetzung, und
0,09 Gt Triphenylsulfonium-hexafluorantimonat und
7,5 Gt Propylenglykol-monomethylether-acetat.

Von dieser Lösung werden 2,0 ml durch ein Filter mit einem Porendurchmesser von 0,2 µm filtriert und auf einen Siliciumwafer, der mit Hexamethyldisilazan als Haftvermittler überzogen ist, aufgetragen. Die Schleuderdrehzahl wird dabei so gewählt, daß man nach 40 s eine homogene Schicht mit einer Schichtdikke von etwa 1 µm erhält. Der Wafer wird auf einer Heizplatte bei 100 °C 60 s lang getrocknet, anschließend mit einer bildmäßig strukturierten Testmaske in Kontakt gebracht und unter Verwendung eines KrF-Excimer-Lasers mit Strahlung von 248 nm bestrahlt (70 $mJ/cm^2$). Danach wird der Wafer 1 min bei 90 °C gehalten und mit Anisol 120 s entwickelt. Strukturen von 0,70 µm (line/space) werden aufgelöst.

Anwendungsbeispiel 5:

Es wird eine Lösung eines strahlungsempfindlichen Gemisches hergestellt aus
2,5 Gt des Polymeren 7b, entsprechend der oben angegebenen Zusammensetzung, und
0,075 Gt Triphenylsulfonium-trifluormethansulfonat und
7,5 Gt Propylenglykol-monomethylether-acetat.

Von dieser Lösung werden 2,0 ml durch ein Filter mit einem Porendurchmesser von 0,2 µm filtriert und auf einen Siliciumwafer, der mit Hexamethyldisilazan als Haftvermittler überzogen ist, aufgetragen. Die Schleuderdrehzahl wird dabei so gewählt, daß man nach 40 s eine homogene Schicht mit einer Schichtdikke von etwa 1 µm erhält. Der Wafer wird auf einer Heizplatte bei 100 °C 60 s lang getrocknet, anschließend mit einer bildmäßig strukturierten Testmaske in Kontakt gebracht und unter Verwendung eines KrF-Excimer-Lasers mit Strahlung der Wellenlänge 248 nm bestrahlt (55 $mJ/cm^2$). Danach wird der belichtete Wafer 1 min einem post exposure bake von 85 °C unterzogen und mit einer 0,135 n wäßrigen Tetramethylammoniumnydroxid-Lösung 45 s entwickelt. Strukturen von 0,55 µm (line/space) werden aufgelöst.

**Patentansprüche**

**1.** Verbindung der allgemeinen Formel II

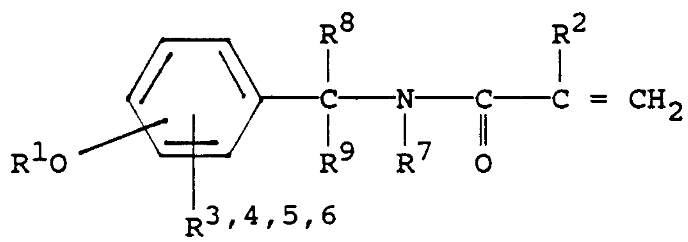

(II)

worin

$R^1$ eine durch Säure abspaltbare Gruppe und

$R^2$ $(C_1-C_4)$Alkyl, Halogen$(C_1-C_4)$alkyl, Halogen, CN oder Wasserstoff bedeutet,

| | |
|---|---|
| $R^{3,4,5,6}$ | voneinander unabhängig einen gegebenenfalls halogen-substituierten aliphatischen, araliphatischen oder aromatischen Rest mit 1 bis 20 Kohlenstoffatomen, Halogen, Hydroxy oder Wasserstoff darstellen und |
| $R^7$ | ein Wasserstoffatom oder eine $(C_1-C_4)$-Alkylgruppe bedeutet, |
| $R^8$ und $R^9$ | gleich oder verschieden sind und Wasserstoff, $(C_1-C_6)$Alkyl- oder $(C_6-C_{10})$-Arylgruppen darstellen. |

**2.** Verbindung gemäß Anspruch 1, dadurch gekennzeichnet, daß

| | |
|---|---|
| $R^1$ | eine Benzyl-, Trialkylsilanyl-, Alkoxycarbonyl-, Tetrahydropyranyl- oder Tetrahydrofuranyl-Gruppe, |
| $R^2$ | Wasserstoff oder eine Methylgruppe ist, |
| $R^3$ bis $R^6$ | unabhängig voneinander jeweils Wasserstoff, eine Hydroxy- oder eine Methylgruppe darstellen, wobei bevorzugt nicht mehr als zwei dieser Reste Methylgruppen sind, und |
| $R^7$ bis $R^9$ | Wasserstoff bedeuten. |

**3.** Verbindung gemäß Anspruch 2, dadurch gekennzeichnet, daß die Gruppe $OR^1$ eine tert.-Butoxycarbonyloxy-Gruppe ist und ortho- oder paraständig zur $-CR^8R^9$-Gruppe steht.

**4.** Polymer mit Einheiten der allgemeinen Formel I

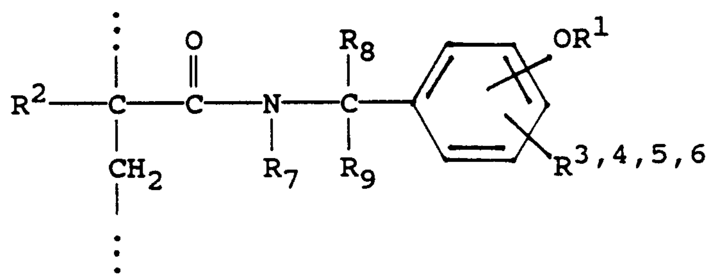

(I)

worin $R^1$ bis $R^9$ die im Anspruch 1 angegebene Bedeutung haben.

**5.** Polymer gemäß Anspruch 4, dadurch gekennzeichnet, daß die Reste $R^1$ bis $R^9$ die im Anspruch 2 angegebene Bedeutung haben.

**6.** Polymer gemäß Anspruch 4, dadurch gekennzeichnet, daß die Gruppe $R^1$ eine tert.-Butoxycarbonyl-Gruppe ist und $OR^1$ sich in ortho- oder para-Stellung befindet.

**7.** Polymer gemäß einem oder mehreren der Ansprüche 4 bis 6, dadurch gekennzeichnet, daß es zu mindestens 10 mol-% aus Einheiten der allgemeinen Formel II und zum Rest aus Einheiten, abgeleitet aus anderen Vinylmonomeren, besteht.

**8.** Polymer gemäß einem oder mehreren der Ansprüche 4 bis 6, dadurch gekennzeichnet, daß es ein Homopolymer ist und neben den Einheiten der allgemeinen Formel I keine weiteren Einheiten enthält.

**9.** Polymer gemäß Anspruch 7, dadurch gekennzeichnet, daß es ein Mischpolymer ist und neben Einheiten der allgemeinen Formel I mit säurespaltbaren Gruppen Einheiten abgeleitet von anderen Vinylmonomeren, vorzugsweise von solchen der allgemeinen Formel II mit $R^1$ = H oder einer nicht säurespaltbaren Gruppe, von Maleimid, von Methacrylsäure, einem Ester oder Amid der (Meth)-acrylsäure und/oder von einem Vinylether, enthält.

**10.** Polymer gemäß einem oder mehreren der Ansprüche 4 bis 9, dadurch gekennzeichnet, daß es ein mittleres Molekulargewicht von 2.000 bis 100.000, bevorzugt 5.000 bis 40.000, aufweist.

**11.** Strahlungsempfindliches Gemisch mit einem polymeren Bindemittel, das durch Säure abspaltbare Seitengruppen aufweist, und einer bei Bestrahlung eine starke Säure bildende Verbindung, dadurch gekennzeichnet, daß als Bindemittel ein Polymer gemäß einem oder mehreren der Ansprüche 4 bis 10

verwendet wird.

**12.** Strahlungsempfindliches Gemisch gemäß Anspruch 11, dadurch gekennzeichnet, daß der Säurebildner ein 1-Sulfonyloxy-2-pyridon, ein Nitrobenzylester, ein Bissulfonyldiazomethan, ein 1,2,3-Tris-(alkylsulfonyloxy- oder -arylsulfonyloxy)-benzol und/oder ein Oniumsalz ist.

**13.** Strahlungsempfindliches Gemisch gemäß Anspruch 11 oder 12, dadurch gekennzeichnet, daß der Anteil des Säurebildners im strahlungsempfindlichen Gemisch 1 bis 40 Gew.-%, insbesondere 2 bis 15 Gew.-%, bezogen auf das Gesamtgewicht der Feststoffe im Gemisch, beträgt.

**14.** Strahlungsempfindliches Gemisch gemäß einem oder mehreren der Ansprüche 11 bis 13, dadurch gekennzeichnet, daß der Anteil des Bindemittels im Gemisch 60 bis 99 Gew.-%, insbesondere 90 bis 97 Gew.-%, bezogen auf das Gesamtgewicht der Feststoffe im Gemisch, beträgt.

**15.** Aufzeichnungsmaterial mit einem Träger und einer darauf befindlichen strahlungsempfindlichen Schicht, dadurch gekennzeichnet, daß die strahlungsempfindliche Schicht aus einem strahlungsempfindlichen Gemisch gemäß einem oder mehreren der Ansprüche 11 bis 14 besteht.

**16.** Verfahren zur Anwendung von Reliefstrukturen oder zur Strukturierung von Wafern durch Auftragen einer Photoresistschicht, die nach dem Trocknen eine Dicke von 0,1 bis 5 µm aufweist, auf ein in üblicher Weise vorbehandeltes Substrat, bildmäßiges Belichten, gegebenenfalls Erhitzen auf Temperaturen bis 150 °C und Entwickeln entweder mit einer wäßrig-alkalischen Lösung für ein positives Bild oder mit einem organischen Lösemittel für ein negatives Bild, dadurch gekennzeichnet, daß die Photoresistschicht ein strahlungsempfindliches Gemisch gemäß einem oder mehreren der Ansprüche 11 bis 15 enthält.

## Claims

**1.** A compound of the formula II

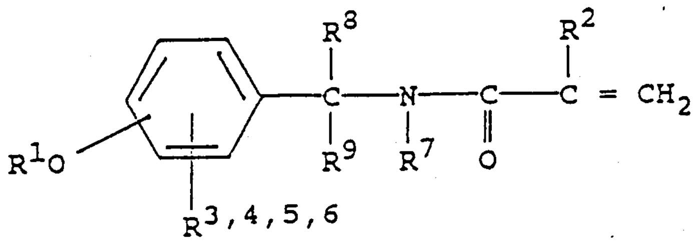

(II)

in which

| | |
|---|---|
| $R^1$ | is an acid-cleavable group, |
| $R^2$ | is $(C_1\text{-}C_4)$-alkyl, halogeno-$(C_1\text{-}C_4)$-alkyl, halogen, CN or hydrogen, |
| $R^{3,4,5,6}$ | independently of one another are optionally a halogen-substituted aliphatic, araliphatic or aromatic radical having 1 to 20 carbon atoms, halogen, hydroxyl or hydrogen, |
| $R^7$ | is a hydrogen atom or a $(C_1\text{-}C_4)$-alkyl group, and |
| $R^8$ and $R^9$ | are identical or different and are hydrogen, a $(C_1\text{-}C_6)$-alkyl group or a $(C_6\text{-}C_{10})$-aryl group. |

**2.** A compound as claimed in claim 1, wherein

| | |
|---|---|
| $R^1$ | is a benzyl, trialkylsilanyl, alkoxycarbonyl, tetrahydropyranyl or tetrahydrofuranyl group, |
| $R^2$ | is hydrogen or a methyl group, |
| $R^3$ to $R^6$ | independently of one another are each hydrogen, a hydroxyl group or a methyl group, preferably not more than two of these radicals being methyl groups, and |
| $R^7$ to $R^9$ | are hydrogen. |

**3.** A compound as claimed in claim 2, wherein the group $OR^1$ is a tert.-butoxycarbonyloxy group in the ortho-or para-position relative to the $-CR^8R^9$ group.

**4.** A polymer with units of the formula I

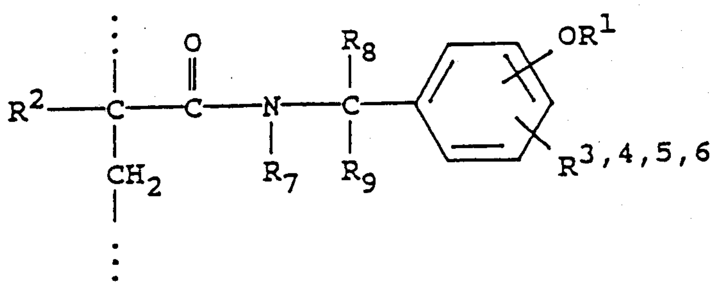

(I)

in which $R^1$ to $R^9$ have the definitions given in claim 1.

**5.** A polymer as claimed in claim 4, wherein the radicals $R^1$ to $R^9$ have the definitions given in claim 2.

**6.** A polymer as claimed in claim 4, wherein the group $R^1$ is a tert.-butoxycarbonyl group and $OR^1$ is in the ortho- or para-position.

**7.** A polymer as claimed in one or more of claims 4 to 6, which comprises at least 10 mol% of units of the formula II, the remainder being composed of units derived from other vinyl monomers.

**8.** A polymer as claimed in one or more of claims 4 to 6, which is a homopolymer and does not contain any further units other than the units of the formula I.

**9.** A polymer as claimed in claim 7, which is a copolymer and, in addition to units of the formula I with acid-cleavable groups, contains units derived from other vinyl monomers, preferably those of the formula II with $R^1$ = H or a non-acid-cleavable group, or from maleimide, from methacrylic acid or an ester or amide of (meth)acrylic acid and/or from a vinyl ether.

**10.** A polymer as claimed in one or more of claims 4 to 9, which has an average molecular weight of 2,000 to 100,000, preferably 5,000 to 40,000.

**11.** A radiation-sensitive mixture with a polymeric binder having acid-cleavable side groups,
and with a compound which generates a strong acid on irradiation, wherein the binder used is a polymer as claimed in one or more of claims 4 to 10.

**12.** A radiation-sensitive mixture as claimed in claim 11, wherein the acid generator is a 1-sulfonyloxy-2-pyridone, a nitrobenzyl ester, a bis-sulfonyldiazomethane, a 1,2,3-tris-(alkylsulfonyloxy- or - arylsulfonyloxy)-benzene and/or an onium salt.

**13.** A radiation-sensitive mixture as claimed in claim 11 or 12, wherein the proportion of the acid generator in the radiation-sensitive mixture is 1 to 40% by weight, in particular 2 to 15% by weight, relative to the total weight of the solids in the mixture.

**14.** A radiation-sensitive mixture as claimed in one or more of claims 11 to 13, wherein the proportion of the binder in the mixture is 60 to 99% by weight, in particular 90 to 97% by weight, relative to the total weight of the solids in the mixture.

**15.** A recording material with a support and a radiation-sensitive layer located thereon, wherein the radiation-sensitive layer is composed of a radiation-sensitive mixture as claimed in one or more of claims 11 to 14.

**16.** A method of using relief structures or of structuring wafers by applying a photoresist layer which, after drying, has a thickness of 0.1 to 5 µm, to a conventionally pretreated substrate, imagewise exposure, heating to temperatures of up to 150 °C if appropriate, and developing either with an aqueous-alkaline solution for a positive image or with an organic solvent for a negative image, wherein the photoresist

layer contains a radiation-sensitive mixture as claimed in one or more of claims 11 to 15.

**Revendications**

**1.** Composé de formule générale II

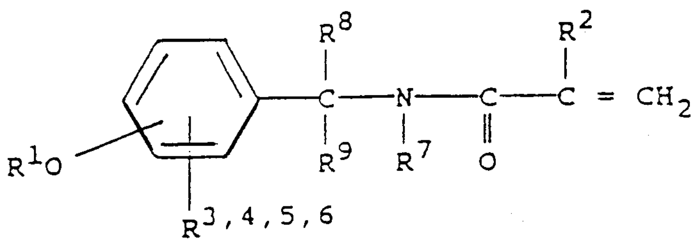

(II)

dans laquelle

- $R^1$ représente un groupe séparable par un acide et
- $R^2$ représente un atome d'hydrogène ou d'halogène ou un groupe alkyle en $C_1$-$C_4$, halogénoalkyle en $C_1$-$C_4$ ou CN,
- $R^{3,4,5,6}$ représentent, indépendamment les uns des autres, un radical aliphatique, araliphatique ou aromatique, éventuellement substitué par des atomes d'halogène, ayant de 1 à 20 atomes de carbone, un atome d'hydrogène ou d'halogène ou le groupe hydroxy, et
- $R^7$ représente un atome d'hydrogène ou un groupe alkyle en $C_1$-$C_4$,
- $R^8$ et $R^9$ sont identiques ou différents et représentent un atome d'hydrogène ou un groupe alkyle en $C_1$-$C_6$ ou aryle en $C_6$-$C_{10}$.

**2.** Composé selon la revendication 1, caractérisé en ce que

- $R^1$ représente un groupe benzyle, trialkylsilanyle, alcoxycarbonyle, tétrahydropyrannyle ou tétrahydrofurannyle,
- $R^2$ est un atome d'hydrogène ou le groupe méthyle,
- $R^3$ à $R^6$ représentent chacun, indépendamment les uns des autres, un atome d'hydrogène ou le groupe hydroxy ou méthyle, de préférence pas plus de deux de ces radicaux n'étant des groupes méthyle, et
- $R^7$ à $R^9$ représentent des atomes d'hydrogène.

**3.** Composé selon la revendication 2, caractérisé en ce que le groupe $OR^1$ est un groupe tert-butoxycarbonyloxy et se trouve en position ortho ou para par rapport au groupe $-CR^8R^9$.

**4.** Polymère à motifs de formule générale I

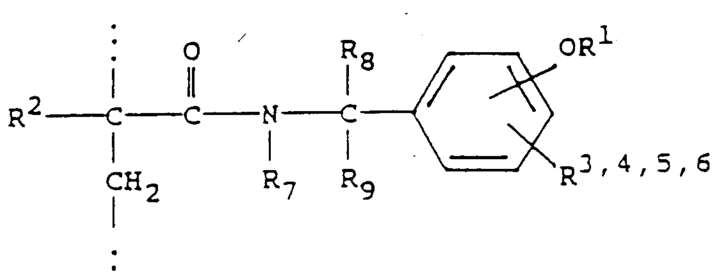

(I)

dans laquelle $R^1$ à $R^9$ ont les significations données dans la revendication 1.

**5.** Polymère selon la revendication 4, caractérisé en ce que les radicaux $R^1$ à $R^9$ ont les significations données dans la revendication 2.

**6.** Polymère selon la revendication 4, caractérisé en ce que le groupe $R^1$ est un groupe tert-butoxycarbonyle, et $OR^1$ se trouve en position ortho ou para.

**7.** Polymère selon une ou plusieurs des revendications 4 à 6, caractérisé en ce qu'il consiste à raison d'au moins 10 % en moles en motifs de formule générale II et, pour le reste, en motifs dérivés d'autres monomères vinyliques.

**8.** Polymère selon une ou plusieurs des revendications 4 à 6, caractérisé en ce qu'il est un homopolymère et ne contient pas d'autres motifs, en plus des motifs de formule générale I.

**9.** Polymère selon la revendication 7, caractérisé en ce qu'il est un copolymère et contient, en plus de motifs de formule générale I à groupes séparables par un acide, des motifs dérivés d'autres monomères vinyliques, de préférence de ceux de formule générale II dans lesquels $R^1$ = H ou d'un groupe non séparable par un acide, du maléimide, de l'acide méthacrylique, d'un ester ou amide de l'acide (méth)acrylique et/ou d'un éther vinylique.

**10.** Polymère selon une ou plusieurs des revendications 4 à 9, caractérisé en ce qu'il présente une masse moléculaire moyenne de 2 000 à 100 000, de préférence de 5 000 à 40 000.

**11.** Composition sensible aux radiations, comportant un liant polymère qui présente des groupes latéraux séparables par un acide, et un composé formant à l'irradiation un acide fort, caractérisée en ce que, comme liant, on utilise un polymère selon une ou plusieurs des revendications 4 à 10.

**12.** Composition sensible aux radiations selon la revendication 11, caractérisée en ce que le générateur d'acide est une 1-sulfonyloxy-2-pyridone, un ester nitrobenzylique, un bisulfonyldiazométhane, un 1,2,3-tris-(alkylsulfonyloxy- ou -arylsulfonyloxy)benzène et/ou un sel d'onium.

**13.** Composition sensible aux radiations selon la revendication 11 ou 12, caractérisée en ce que la proportion du générateur d'acide dans la composition sensible aux radiations est de 1 à 40 % en poids, en particulier de 2 à 15 % en poids, par rapport au poids total des matières sèches contenues dans la composition.

**14.** Composition sensible aux radiations selon une ou plusieurs des revendication 11 à 13, caractérisée en ce que la proportion du liant dans la composition est de 60 à 99 % en poids, en particulier de 90 à 97 % en poids, par rapport au poids total des matières sèches contenues dans le mélange.

**15.** Matériau de reprographie comportant un support et une couche sensible aux radiations se trouvant sur celui-ci, caractérisé en ce que la couche sensible aux radiations consiste en une composition sensible aux radiations selon une ou plusieurs des revendications 11 à 14.

**16.** Procédé pour l'application de motifs en relief ou pour le modelage de plaquettes par application d'une couche de photorésist qui présente, après le séchage, une épaisseur de 0,1 à 5 µm, sur un substrat prétraité à la manière usuelle, insolation selon l'image, éventuellement chauffage à des températures allant jusqu'à 150 °C, et développement soit avec une solution aqueuse-alcaline pour une image positive, soit avec un solvant organique pour une image négative, caractérisé en ce que la couche de photorésist contient une composition sensible aux radiations selon une ou plusieurs des revendications 11 à 15.